# EUROPEAN PATENT APPLICATION

(11) **EP 4 653 035 A1**
(43) Date of publication of application: **26.11.2025**
(21) Application number: 24893061.2
(22) Date of filing: 08.10.2024
(51) Int. Cl.: A61M 25/00, A61M 25/14, A61F 2/04

(54) **ANTI-REFLUX URETERAL STENT**

(30) Priority: 23.11.2023 CN 202311568866
(71) Applicant: Nanchang Chengxi Techonology Equipment Co., Ltd., Nanchang, Jiangxi 330000 (CN)
(72) Inventor: KUANG, Renrui, Nanchang, Jiangxi 330000 (CN)
(74) Representative: Hirsch & Associés
(86) International application number: PCT/CN2024/123296
(87) International publication number: WO 2025/107902

(57) **Abstract**

The present invention provides an anti-reflux ureteral stent. A leaflet-shaped structure is arranged on a side wall of the stent at a bladder end to achieve a one-way drainage function, and the leaflet-shaped structure defines a lumen of the stent in a cross-section along with a tube wall of the stent. The outer surface of the leaflet-shaped structure is larger than the inner surface thereof. When the external pressure of the leaflet-shaped structure is larger than the internal pressure of the leaflet-shaped structure, the leaflet-shaped structure is attached to the tube wall, and the tube wall supports the leaflet-shaped structure, thereby sealing the lumen. The present invention can achieve, by means of the leaflet-shaped structure, one-way drainage in the ureteral stent, so as to effectively achieve an anti-reflux effect.

## Description

### TECHNICAL FIELD

The present disclosure relates to the technical field of medical apparatus and instruments, and in particular to an anti-reflux ureteral stent.

### BACKGROUND

A ureter is a tubular organ that connects the kidney to the bladder, and serves to deliver urine from the kidney to the bladder. A ureteral stent is a supporting structure placed into the ureter, is mainly of a tubular structure, and may also be of a grid-like structure, a T-shaped structure and many other variants. The ureteral stent is provided with retaining structures at two ends of the pelvis and the bladder. These retaining structures are usually formed by curling tubular structures. There are also retaining structures that take the special shapes of balloons, trumpets, umbrellas, coils, barbs, etc. The retaining structures avoid displacement of a stent tube and are important structures to ensure function realization of the ureteral stent.

For endoluminal surgeries of the kidney and the ureter, postoperative indwelling of the ureteral stent tube is a routine way of treatment. Millions of ureteral stents are placed into human bodies every year, with the indwelling time varying from a few days to several months. However, most of these patients experience varying degrees of postoperative discomfort, primarily urinary tract irritation and pain, which affect the patients' state of life and ability to work. Current studies suggest that a significant cause of these discomforts is reflux of urine when bladder pressure rises.

There are three main solutions for reducing bladder-pelvis reflux, i.e., designing various one-way valve structures inside a lumen of a ureteral stent, as described in the patent with the authorization announcement No. CN206403899U, and arranging a one-way valve at an end of a ureteral stent, as described in the patent with the authorization announcement No. CN204563249U, and achieving the function using an anti-reflux structure of a bladder and ureter connecting portion of a human body by placing a tubular structure of the ureteral stent inside the ureter outside the wall of the bladder. It is difficult to implement the one-way valve inside a lumen (less than 2 mm) of a small ureteral stent (as described in the patent with the authorization announcement No. CN206403899U), and in case of indwelling for a long time, precipitation of urine components in the one-way valve may cause the one-way valve to fail in function. In addition, the one-way valve may also hinder entry of a guide wire, making it difficult to place the ureteral stent, and the passage of the guide wire may affect the structure and the function of the one-way valve. Limiting the stent outside a bladder wall section of the ureter can take advantage of the anti-reflux structure of the human body, but at the same time, may lead to an incomplete supporting function of the stent, which may result in poor urine drainage from the kidney and complications such as back pain and fever. Moreover, it is difficult to keep the exact position of the ureteral stent, and the stent tube may move up, resulting in an inaccurate drainage effect. Adding an additional one-way valve to an end of the ureteral stent (as described in the patent with the authorization announcement No. CN204563249U) results in an increase in length and volume of the stent, an increase in irritation symptoms of the stent tube, and a tendency of displacement of the stent tube with urine during urination, which makes it difficult to be widely used.

In summary, the ureteral stent that is currently widely used in clinical practice has the problem of reflux, which is likely to cause discomfort to patients. However, since the ureteral stent is relatively small, it is difficult to provide an anti-reflux structure, and the anti-reflux structure of the human body will lead to an incomplete supporting function of the ureteral stent, which in turn makes it difficult to solve the problem of reflux of the ureteral stent. At present, there is no satisfactory solution that can achieve a significant anti-reflux effect while maintaining the function of the stent.

### SUMMARY

Based on this, an objective of some embodiments to provide an anti-reflux ureteral stent. The anti-reflux ureteral stent mainly achieves an anti-reflux function by means of a flap-like structure on a side wall of a stent tube. In this solution, the form of the stent tube is not changed, such that additional irritation may not be caused to a bladder, the ability for a guide wire to pass through a lumen may not be affected by the flap-like structure on the side wall of the stent tube, and the drainage ability of the stent tube may not be interfered, either. It is easy to implement the solution so as to overcome shortcomings in the conventional technology.

The present disclosure provides the following technical solutions, including:
a catheter body;
a first end and a second end, the first end and the second end being respectively arranged at two ends of the catheter body, wherein
the first end comprises a first end portion and a first retaining structure for being arranged in a kidney, the first retaining structure is arranged at one end of the catheter body, the first end portion is arranged at an end portion of the first retaining structure away from the catheter body, and the first end portion being provided with an opening;
the second end comprises a second end portion and a second retaining structure for being arranged in a bladder, the second retaining structure being arranged at an end of the catheter body away from the first retaining structure, the second end portion is arranged at an end portion of the second retaining structure away from the catheter body, the second retaining structure and a part of the catheter body within two centimeters from the second retaining structure are both provided with one or more flap-like structures, and the flap-like structures are arranged on a side face of a tube wall of the anti-reflux ureteral stent but not at the second end portion;
the flap-like structures and the tube wall of the anti-reflux ureteral stent jointly enclose a lumen of the anti-reflux ureteral stent in cross-section; an outer surface of each of the flap-like structures is greater than an inner surface of each of the flap-like structures; when an external pressure of the flap-like structures is higher than an internal pressure of the flap-like structures, the flap-like structure closely fits the tube wall, and the tube wall supports the flap-like structures to realize closure of the lumen; and when the external pressure of the flap-like structures is lower than the internal pressure of the flap-like structures, the flap-like structures are pushed open to realize opening of the lumen.

In an embodiment, both of the first retaining structure and the second retaining structure are of curled structures, and the flap-like structure is arranged on an inner side of the second retaining structure.

In an embodiment, the flap-like structures are arranged at a quarter of the second retaining structure adjacent to the second end portion.

In an embodiment, ends of the flap-like structures adjacent to the second end portion gradually narrow.

In an embodiment, the flap-like structures are bonded to an outer wall of the second retaining structure by a water-soluble polymeric material.

In an embodiment, an outer wall of each of the flap-like structures is provided with a recess.

In an embodiment, the catheter body and the flap-like structure are made of different materials.

In an embodiment, the first end and a side wall of the catheter body are provided with a plurality of side holes.

In an embodiment, the recess is a transverse recess.

Compared with the conventional technology, some embodiments have the following beneficial effects. According to some embodiments, the flap-like structure may be implemented by adding a simple cutting process based on an existing stent production process, which basically does not increase the production cost and is highly feasible. In addition, the form of the stent is not changed, so that the anti-reflux function can be achieved without adding side effects. There is no need to change the mode of operation by a surgeon when the stent according to the present disclosure is in use, so that additional learning costs or risks may not be brought to a surgery. That is, by applying the present disclosure, an anti-reflux effect can be achieved without increasing the cost, generating side effects and changing operation habits. The first end and the second end are arranged at the two ends of the catheter body, the first end includes the first retaining structure, and the second end includes the second retaining structure. In practice, the first end is located inside a pelvis, the second retaining structure and a part of the catheter body about two centimeters from the second retaining structure are located inside the bladder, and the flap-like structure is provided on the stent inside the bladder. When the external pressure of the flap-like structure is lower than the internal pressure, i.e., when the external pressure of a bladder section of the stent is lower than the internal pressure, the internal pressure may push the flap-like structure open, which in turn causes a fluid inside to flow smoothly, thereby realizing smooth drainage from the kidney to the bladder. When the external pressure of the flap-like structure is higher than the internal pressure, i.e., when the external pressure of the stent is higher than the internal pressure, the flap-like structure abuts against the tube wall of the stent, and the tube wall of the stent supports the flap-like structure. Since the second end portion is not provided with an opening for the fluid to flow, the lumen is isolated from the outside after the flap-like structure closely fits the tube wall, thereby preventing an external fluid from entering the stent and preventing urine from flowing back to the kidney. According to an experiment in which the above-described anti-reflux stent and an ordinary ureteral stent are placed in the same normal saline bottle at the same height, compared with the normal ureteral stent, the anti-reflux stent can completely eliminate reflux.

### BRIEF DESCRIPTION OF THE DRAWINGS

FIG. 1 is a schematic structural diagram of an anti-reflux ureteral stent according to an embodiment of the present disclosure;
FIG. 2 is a schematic structural diagram of a second retaining structure of the anti-reflux ureteral stent according to an embodiment of the present disclosure;
FIG. 3 is a first sectional view of a flap-like structure of the anti-reflux ureteral stent according to an embodiment of the present disclosure;
FIG. 4 is a second sectional view of the flap-like structure of the anti-reflux ureteral stent according to an embodiment of the present disclosure; and
FIG. 5 is a sectional view of a second end portion of the anti-reflux ureteral stent according to an embodiment of the present disclosure.

Reference signs of the main elements:
10 catheter body;
20 first end; 21 first end portion; 22 first retaining structure;
30 second end; 300 side hole; 31 second end portion; 32 second retaining structure; 33 flap-like structure; 331 transverse recess; 332 inner surface; 333 outer surface; 334 incision;
40 guide wire.

The present disclosure will be further described in the following detailed description of the embodiments with reference to the above drawings.

### DETAILED DESCRIPTION OF THE EMBODIMENTS

In order to facilitate understanding of the present disclosure, the present disclosure will be described more fully below with reference to the relevant accompanying drawings. Several embodiments of the present disclosure are given in the accompanying drawings. However, the present disclosure may be implemented in many different forms and is not limited to the embodiments described herein. Rather, these embodiments are provided to make the content of the present disclosure more thorough and comprehensive.

It should be noted that when an element is said to be "fixed" to another element, it may be directly on another element or there may also be an element therebetween. When an element is said to be "connected" to another element, it may be directly connected to another element or there may also be an element therebetween at the same time. The terms "vertical", "horizontal", "left", "right" and similar expressions are used herein for illustrative purposes only.

Unless otherwise defined, all technical and scientific terms used herein shall have the same meanings as commonly understood by those skilled in the art to which the present disclosure belongs. The terms used in the specification of the present disclosure are merely for the purpose of describing specific examples, but are not intended to limit the present disclosure. As used herein, the term "and/or" includes any or all combinations of one or more of the relevant listed items.

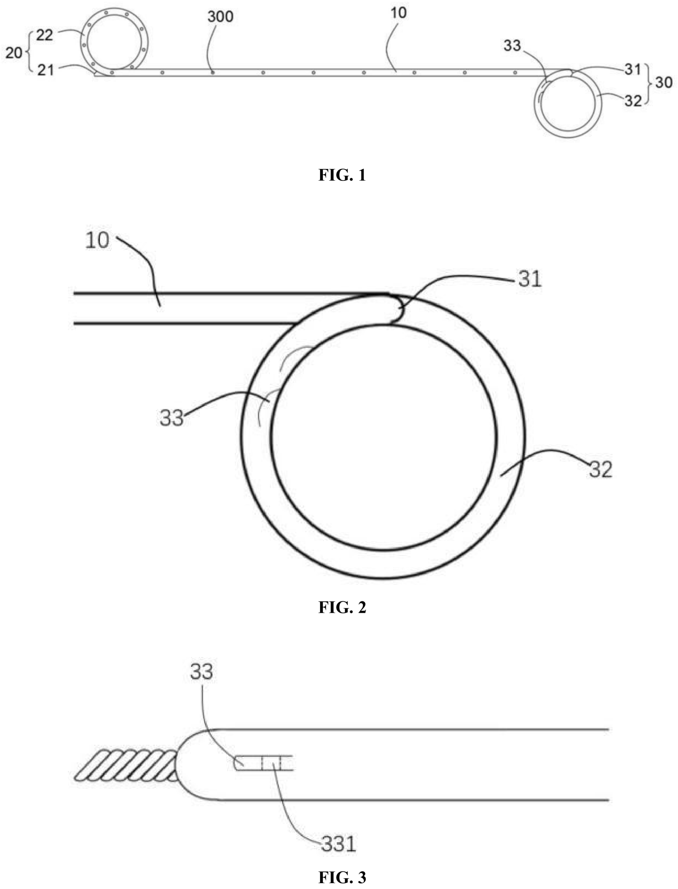

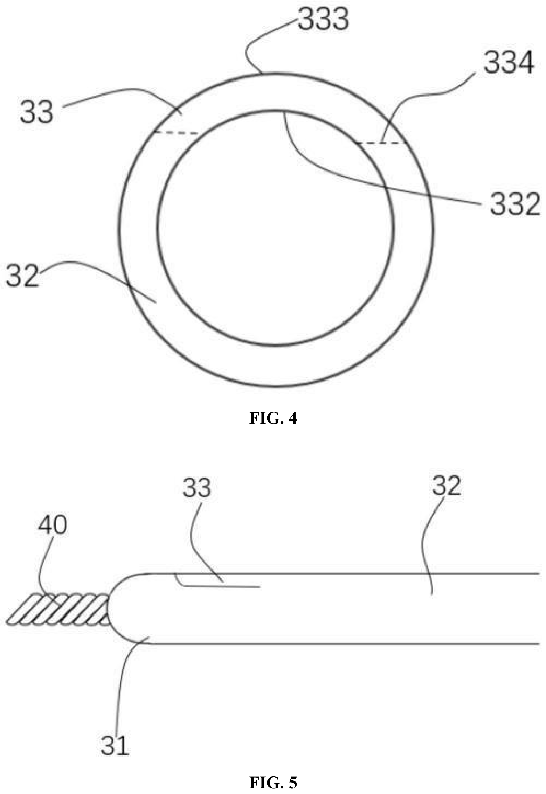

Referring to FIGs. 1 to 5, an anti-reflux ureteral stent according to an embodiment of the present disclosure is shown, including a catheter body 10, a first end 20 and a second end 30.

The first end 20 is configured to be arranged inside a kidney, and the second end 30 is configured to be arranged inside a bladder. The first end 20 and the second end 30 are respectively arranged at two ends of the catheter body 10. The first end 20 includes a first end portion 21 and a first retaining structure 22 for being arranged in the kidney. The first retaining structure 22 is arranged at one end of the catheter body 10. The first end portion 21 is arranged at an end portion of the first retaining structure 22 away from the catheter body 10. The first end portion 21 is provided with an opening. The second end 30 includes a second end portion 31 and a second retaining structure 32 for being arranged in the bladder. The second retaining structure 32 is arranged at an end of the catheter body 10 away from the first retaining structure 22. The second end portion 31 is arranged at an end portion of the second retaining structure 32 away from the catheter body 10. Neither the second end 30 nor a part of a stent main body located inside the bladder is provided with other openings for a fluid to flow other than the flap-like structure 33, and only the second end portion 31 is provided with a puncture or an incision through which a guide wire can pass. The puncture or the incision is closed in a natural state. Specifically, the second end portion 31 has no opening for the fluid to flow, but only a puncture for the guide wire to pass through. The second retaining structure 32 and a part of the catheter body 10 within two centimeters from the second retaining structure 32 are both provided with one or more flap-like structures 33. The flap-like structures 33 are arranged on a side face of a tube wall but not at the second end portion 31. The flap-like structure 33 and the tube wall of the stent jointly enclose a lumen of the stent in cross-section. An outer surface 333 of the flap-like structure 33 is greater than an inner surface 332 of the flap-like structure 33. When an external pressure of the flap-like structure 33 is higher than an internal pressure of the flap-like structure 33, the flap-like structure 33 closely fits the tube wall, and the tube wall supports the flap-like structure 33 to realize closure of the lumen. When the external pressure of the flap-like structure 33 is lower than the internal pressure of the flap-like structure 33, the flap-like structure 33 is pushed open to realize opening of the lumen.

Specifically, the second retaining structure 32 and the side face of the tube wall of the catheter body 10 are both provided with the plurality of flap-like structures 33. The flap-like structures 33 enclose the lumen of the stent together with the second retaining structure 32 and the tube wall of the catheter main body 10 in cross-section.

It can be understood that the catheter body 10 is now arranged inside a ureter, the first end 20 and the second end 30 are respectively arranged inside the kidney and the bladder, and urine, after being produced, flows to the second end 30 due to peristalsis of smooth muscle of a pelvis. Pressure inside the bladder is lower than pressure inside the flap-like structure 33 when pressure of urine inside the second retaining structure 32 is higher than pressure of urine inside the bladder. At the moment, the flap-like structure 33 is pushed open to open the lumen, and urine flows out of the catheter body 10. When the pressure inside the bladder is higher than the pressure inside the flap-like structure 33, the flap-like structure 33 closely fits the tube wall structure of the second end 30 or the catheter body, the tube wall structure of the second end 30 or the catheter body supports the flap-like structure 33, the flap-like structure 33 cannot deform toward the inside of the lumen, and the lumen is closed, so that urine cannot flow into the second retaining structure 32. Since the second end portion 31 is not provided with an opening for the fluid to flow but only provided with the puncture for the guide wire to pass through, urine cannot flow into the second retaining structure 32, thereby achieving the anti-reflux function and improving the patient's comfort of use.

In this embodiment, the flap-like structure 33 and the second end 30 or the tube wall structure of the catheter body 10 jointly enclose the lumen of the stent in cross-section. The outer surface 333 of the flap-like structure 33 is greater than the inner surface 332 of the flap-like structure 33 of the lumen, and sufficient structural strength is retained, so that the structure of the lumen can be maintained. When the external pressure of the flap-like structure 33 is higher than the internal pressure of the flap-like structure 33, the flap-like structure 33 closely fits the tube wall structure of the second end 30 or the catheter body 10, the tube wall structure of the second end 30 or the catheter body 10 supports the flap-like structure 33, the flap-like structure 33 cannot deform toward the inside of the lumen, and closure of the lumen is realized. When the external pressure of the flap-like structure 33 is lower than the internal pressure of the flap-like structure 33, the flap-like structure 33 is pushed open to realize opening of the lumen, thereby realizing a unidirectional drainage effect.

It is worth noting that the flap-like structure 33 may be formed by cutting a tubular structure of an original stent tube, or the flap-like structure 33 may also be added after the tube wall of the stent tube is partially removed. Pressure required for opening the flap-like structure 33 may be adjusted by adjusting the thickness and the size of a root portion of the flap-like structure 33. The drainage capacity of the flap-like structure 33 can be adjusted by adjusting the length and the size of the flap-like structure 33. The incision 334 may be L-shaped, sled-shaped, arc-shaped, or the like.

During specific implementation, when the patient urinates, the pressure inside the bladder rises to press the flap-like structure 33 against an outer wall of the second retaining structure 32, so that urine cannot flow back to the kidney from the inside of the catheter. During drainage of the kidney, a worker cuts out an opening in the first end portion 21, so that the opening at the first end portion 21 may allow urine to enter the first retaining structure 22 and to enter the second retaining structure 32 through the catheter body 10. At the moment, the pressure of the catheter body 10 is higher than the external pressure due to the drained urine inside the catheter body 10, i.e., the internal pressure of the flap-like structure 33 is higher than the external pressure, so that the internal pressure pushes the flap-like structure 33 open, which in turn allows urine to enter the bladder smoothly.

It can be understood that a plurality of side holes 300 are formed at the first end 20, i.e., on a side wall of the first retaining structure 22 and a side wall of the catheter body 10, so as to facilitate drainage of urine.

In some optional embodiments, the first retaining structure 22 is of a curled structure.

It should be noted that the first retaining structure 22 is arranged inside the kidney, and at the moment, an end of the catheter body 10 connected to the first retaining structure is also partially arranged inside the kidney. The second retaining structure 32 is arranged inside the bladder, and the catheter body 10 connected to the second retaining structure 32 is also partially arranged inside the bladder. Therefore, the flap-like structure 33 may be arranged at an end of the catheter body 10 adjacent to the second retaining structure 32 or on the second retaining structure 32.

It should be explained that the catheter body 10, the first end portion 21, the first retaining structure 22, the second end portion 31, the second retaining structure 32 and the flap-like structure 33 are integrally molded. Regarding the second retaining structure 32 as a circular ring, the flap-like structure 33 is located on an inner ring of the circular ring or on the catheter body 10 within two centimeters from the second retaining structure.

In some optional embodiments, the second retaining structure 32 is of a curled structure, and the flap-like structure 33 is arranged on an inner side of the curled structure, i.e., the flap-like structure 33 is arranged on an inner side of the second retaining structure 32. In this way, the integrity of the outside tube wall is maintained, which in turn the longitudinal strength of the stent tube is maintained. When the stent tube is pushed into the body along the guide wire, wrinkling of the stent tube, deformation of the lumen, and difficulties in pushing the stent tube in by holding the guide wire can be avoided.

In practice, the ureteral stent needs to sleeve the guide wire and to be pushed forward to a proper position by a jacking tube that also sleeves the guide wire. The stent tube cannot be placed in a conventional manner when improvement of the stent tube reduces the longitudinal strength. In an experiment, when the flap-like structure 33 is arranged on an outer side of the curled structure, the stent tube may be wrinkled as the stent tube is pushed into the body along the guide wire, so that the lumen may deform to wrap the guide wire, and the friction may be increased to make it difficult to continue to push the stent tube in. Providing the flap-like structure 33 on an inner side of the curled structure can avoid a decrease in longitudinal strength and ensure that the stent tube can slide smoothly along the guide wire.

It is worth noting that the second retaining structure 32 is provided with a plurality of flap-like structures 33. In this embodiment, two flap-like structures 33 are provided and arranged on the outer wall of the second retaining structure 32, and the flap-like structures 33 are arranged at a quarter of the second retaining structure 32 adjacent to the second end portion 31. This part is less affected by contraction of the bladder, and the flap-like structures 33 aremore likely to move with the second end portion 31 when the bladder contracts, which further reduces deformation of the flap-like structures 33 caused by the contraction of the bladder and also reduces the influence of the deformation on the anti-reflux capability. In an experiment, the flap-like structure 33 away from the second end portion 31 and close to the catheter body 10 always fails to cling to the tube wall when squeezed, and thus water may flow out of a gap between the tube wall and the flap-like structure 33, reducing the anti-reflux capability.

No drainage side holes are provided within two centimeters from the junction of the catheter body 10 and the second retaining structure 32 and at the second end portion 30, so that the fluid is prevented from flowing back from the drainage side holes.

Specifically, the second end portion 31 is provided with a puncture or an incision 334, which allows the guide wire 40 to pass through only during placement of the ureteral stent and is naturally closed when there is no guide wire 40 so as to prevent the fluid from flowing back through the puncture or the incision. It should be explained that since the second end portion 31 is arranged at one end of the second retaining structure 32, and the flap-like structure 33 is arranged on the second retaining structure 32, when the flap-like structure 33 is pushed open, the effect of drainage can be achieved, and thus the puncture of the second end portion 31 does not need to allow the fluid to pass through.

Specifically, a side wall of the first end 20 and a side wall of the catheter body 10 are both provided with a plurality of side holes 300.

Specifically, an end of the flap-like structure 33 adjacent to the second end portion 31 gradually narrows. It can be understood that the flap-like structure 33 is formed by cutting out the flap-like structure 33 in the outer wall of the second retaining structure 32 by means of side-cutting, so that the flap-like structure 33 has one end gradually narrowing so as to better fit the outer wall of the second retaining structure 32.

In some optional embodiments, one end of the flap-like structure 33 connected to the second retaining structure 32 gradually narrows, with the width ranging from 1/6 to 1/5 of the circumference of the outer wall of the second retaining structure, and the length of the flap-like structure is about 5 times of the width of the flap-like structure, so that a free end of the flap-like structure 33 has less mass and the function of drainage can be completed more easily.

Specifically, the free end of the flap-like structure 33 may point toward the first end portion 21 for ease of placement from the kidney toward the bladder, and may also point toward the second end portion 31 for ease of placement from the bladder toward the kidney.

In some optional embodiments, the flap-like structure 33 with the consistent width can also achieve the anti-reflux effect.

In some optional embodiments, an end of the flap-like structure 33 adjacent to the second end portion 31 gradually narrows, then widens and finally narrows again, which makes it more convenient to push the flap-like structure 33 open.

It can be understood that since the flap-like structure 33 is adjacent to the second end portion 31, during specific implementation, the second end portion 31 is free. This part is less affected by contraction of the bladder, and the flap-like structure 33 is more likely to move with the second end portion 31 when the bladder contracts, which further reduces deformation of the flap-like structure 33 caused by the contraction of the bladder and also reduces the influence of the deformation on the anti-reflux capability.

In some optional embodiments, the flap-like structure 33 is bonded to the outer wall of the second retaining structure 32 by means of a water-soluble polymeric material. It can be understood that the flap-like structure 33 is bonded to the outer wall of the second retaining structure 32 by means of the water-soluble polymer material when the anti-reflux ureteral stent is placed inside the body of the patient, thereby facilitating placement inside the human body, and the water-soluble polymer material may be dissolved by urine produced by the human body, so that the flap-like structure 33 can perform the functions of opening and closing, which in turn enables the flap-like structure 33 to play the anti-reflux role. In this embodiment, the water-soluble polymeric material is fibrin. The direction of the flap-like structure may also be adjusted to respectively adapt to the placement along a direction from the bladder to the kidney and the placement along a direction from the kidney to the bladder, and convenient placement into the body may also be realized without using a bonding material.

It is worth noting that an outer wall of the flap-like structure 33 is provided with a transverse recess 331, and further there are a plurality of transverse recesses 331 on the outer wall of the flap-like structure 33. In this embodiment, there is one transverse recess 331 on an inner wall of the flap-like structure 33. With this arrangement, a pressure difference required for opening the flap-like structure 33 can be effectively reduced, so that the flap-like structure 33 can better play the role of drainage.

In some optional embodiments, the catheter body 10 and the flap-like structure 33 are made of different materials. With this arrangement, the pressure difference required for opening or closing the flap-like structure can be effectively reduced.

In some optional embodiments, the catheter body 10, the first end 20 and second end 30 are all made of polyurethane or silica gel. It is worth noting that the catheter body 10, the first end portion 21, the first retaining structure 22, the second end portion 31, the second retaining structure 32 and the flap-like structure 33 are all made of polyurethane or silica gel.

It should be noted that the catheter body 10 and the flap-like structure 33 are made of different materials. In this embodiment, the catheter body 10 is made of polyurethane, and the flap-like structure 33 is made of silica gel.

The effectiveness of the solutions of the present disclosure was confirmed by the following experiments.

Materials: Ureteral stent tube blanks of the same material (polyurethane) as commercial stent tubes were supplied by ureteral stent manufacturers, and the blanks were not provided with side holes, were closed at one end and open at the other end, and had a peripheral diameter of 6 mm. A tube wall was cut at the closed end of the blank which is of curled structure to form 6 mm * 1.5 mm flap-like structures, and root portions of the flap-like structures pointed toward the open end of the stent tube to make the anti-reflux stent for testing. The closed end of the same ureteral stent tube blank was opened to simulate a normal ureteral stent.

Test on drainage capability:
The open end of the anti-reflux stent tube for testing and the open end of the simulated normal stent were placed in a 500 ml infusion bag and the other ends separately placed in 100 ml measuring cups, and the drainage capacities of the two stents under 20 cm and 40 cm water column pressures were respectively measured. The volume of drainage of the anti-reflux stent was 90% of that of the normal ureteral stent under the 20 cm water column pressure, and the volume of drainage of the anti-reflux stent was 120% of that of the normal ureteral stent under the 40 cm water column pressure.

### Anti-reflux test:

The flap-like structure end of the anti-reflux stent tube for testing and the open end of the simulated normal stent were placed into a 500 ml infusion bag, and the infusion bag was connected to a pressure gauge. The other ends of the anti-reflux stent tube and the simulated normal stent were separately placed in 100 ml measuring cups, and the drainage capacities of the two stents under 30 cm and 60 cm water column pressures were respectively measured. The anti-reflux stent with the flap-like structures arranged on a stent body and the inner side of the curled structure achieved a complete anti-reflux effect, and the volume of drainage of the stent with the flap-like structures arranged on the side face and the outer side of the curled structure was 10-30% of that of the normal stent.

Anti-reflux experiment in simulated state of bladder contraction:
The flap-like structure end of the anti-reflux stent tube for testing and the open end of the simulated normal stent were placed in a 500 ml infusion bag, the other ends of the anti-reflux stent tube for testing and the simulated normal stent were separately placed in 100 ml measuring cups, and the infusion bag was squeezed under 60 cm water column pressure to cause the ureteral stent to deform, so as to simulate a terminal stage of bladder emptying. A result is as follows. The anti-reflux stent for testing, with the flap-like structure arranged adjacent to an end of the stent, showed the highest stability, and the closer the flap-like structure was to the stent main body, the more susceptible it is to the impact of the compression of the bladder wall, and different degrees of reflux were occurred.

Pushing experiment of stent tube:
Anti-reflux stents for testing, provided with different numbers of flap-like structures at different positions, were prepared, and a puncture was formed in the closed end of the stent tube using a 4-edged needle having a diameter of 1.0 mm to facilitate passage of guide wires. A 0.88 mm guide wire passed through the puncture and run through the entire stent. A jacking tube sleeved the guide wire and was pressed against the puncture end of the stent to push the stent forward. The process of pushing the stent into the body during a surgery was simulated. A result is as follows. When the flap-like structure was arranged on an inner side of a curled segment, the influence on the longitudinal strength of the stent tube was minimized, and the sliding along the guide wire would be smooth under the push of the jacking tube. The flap-like structures at any other positions would cause the longitudinally pushed stent tube to deform, and the friction was increased since the guide wire was gripped, making the stent tube difficult to slide.

In summary, according to the anti-reflux ureteral stent of the above embodiments of the present disclosure, the first end 20 and the second end 30 are arranged at the two ends of the catheter body 10, the first end 20 includes the first retaining structure 22, the second end 30 includes the second retaining structure 32, and the flap-like structure 33 is arranged on the second retaining structure 32. The flap-like structure 33 abuts against the outer wall of the second retaining structure 32 when the external pressure of the catheter body 10 is higher than the internal pressure, so that the external fluid can be prevented from entering the second retaining structure 32. The internal pressure can push the flap-like structure 33 open when the external pressure of the catheter body 10 is lower than the internal pressure, which in turn causes the fluid inside to flow out smoothly, so as to realize unidirectional drainage of the ureteral stent and avoid generating a reflux.

In the description of this specification, descriptions with reference to the terms such as "an embodiment", "some embodiments", "example", "specific example", or "some examples" mean that specific features, structures, materials, or characteristics described with reference to the embodiment or example are included in at least one embodiment or example of the present disclosure. In this specification, schematic description of the above terms does not necessarily refer to the same embodiment or example. Furthermore, the described particular features, structures, materials or characteristics can be integrated with any one or more embodiments or examples in a proper manner.

The above examples merely represent several embodiments of the present disclosure, giving specific and detailed description, but should not be understood as limiting the scope of the present patent of invention thereby. It should be noted that several variations and improvements could also be made by those of ordinary skill in the art without departing from the spirit of the present disclosure, and these all fall within the scope of protection of the present disclosure. Therefore, the scope of protection of the present patent of invention shall be in accordance with the appended claims.

## Claims

1. An anti-reflux ureteral stent, **characterized in** comprising:
a catheter body;
a first end and a second end, the first end and the second end being respectively arranged at two ends of the catheter body, wherein
the first end comprises a first end portion and a first retaining structure for being arranged in a kidney, the first retaining structure is arranged at one end of the catheter body, the first end portion is arranged at an end portion of the first retaining structure away from the catheter body, and the first end portion being provided with an opening;
the second end comprises a second end portion and a second retaining structure for being arranged in a bladder, the second retaining structure being arranged at an end of the catheter body away from the first retaining structure, the second end portion is arranged at an end portion of the second retaining structure away from the catheter body, the second retaining structure and a part of the catheter body within two centimeters from the second retaining structure are both provided with one or more flap-like structures, and the one or more flap-like structures are arranged on a side face of a tube wall of the anti-reflux ureteral stent but not at the second end portion;
the one or more flap-like structures and the tube wall of the anti-reflux ureteral stent jointly enclose a lumen of the anti-reflux ureteral stent in cross-section; an outer surface of each of the one or more flap-like structures is greater than an inner surface of each of the one or more flap-like structures; when an external pressure of the one or more flap-like structures is higher than an internal pressure of the one or more flap-like structures, the one or more flap-like structure closely fits the tube wall, and the tube wall supports the one or more flap-like structures to realize closure of the lumen; and when the external pressure of the one or more flap-like structures is lower than the internal pressure of the one or more flap-like structures, the one or more flap-like structures are pushed open to realize opening of the lumen.

2. The anti-reflux ureteral stent according to claim 1, wherein both of the first retaining structure and the second retaining structure are of curled structures, and the one or more flap-like structure is arranged on an inner side of the second retaining structure.

3. The anti-reflux ureteral stent according to claim 2, wherein the one or more flap-like structures are arranged at a quarter of the second retaining structure adjacent to the second end portion.

4. The anti-reflux ureteral stent according to claim 1, wherein an end of each of the one or more flap-like structures adjacent to the second end portion gradually narrows.

5. The anti-reflux ureteral stent according to claim 1, wherein the one or more flap-like structures are bonded to an outer wall of the second retaining structure by a water-soluble polymeric material.

6. The anti-reflux ureteral stent according to claim 1, wherein an outer wall of each of the one or more flap-like structures is provided with a recess.

7. The anti-reflux ureteral stent according to claim 1, wherein the catheter body and the flap-like structures are made of different materials.

8. The anti-reflux ureteral stent according to claim 1, wherein the first end and a side wall of the catheter body are both provided with a plurality of side holes.

9. The anti-reflux ureteral stent according to claim 6, wherein the recess is a transverse recess.
